# FASCICULE DE BREVET EUROPEEN

(11) **EP 2 568 915 B1**
(45) Date de publication et mention de la délivrance du brevet: **08.04.2020**
(21) Numéro de dépôt: 11718403.6
(22) Date de dépôt: 10.05.2011
(51) Int. Cl.: A61C 1/00, A61C 19/00

(54) **DISPOSITIF D'INTERFAÇAGE ENTRE UN UTILISATEUR ET UN INSTRUMENT CHIRURGICAL OU DENTAIRE**
VORRICHTUNG FÜR DIE HERSTELLUNG EINER SCHNITTSTELLE ZWISCHEN EINEM BENUTZER UND EINEM CHIRURGISCHEN ODER ZAHNMEDIZINISCHEN INSTRUMENT
DEVICE FOR INTERFACING BETWEEN A USER AND A SURGICAL OR DENTAL INSTRUMENT

(30) Priorité: 12.05.2010 EP 10162731
(43) Date de publication de la demande: 20.03.2013
(73) Titulaire: Bien-Air Holding SA, 2500 Bienne 6 (CH)
(72) Inventeur: FROCHAUX, Damien, 2013 Colombier (CH); GALLINA, Marco, 3232 Ins (CH)
(74) Mandataire: Supper, Marc
(86) Numéro de dépôt international: PCT/EP2011/057461
(87) Numéro de publication internationale: WO 2011/141442

(56) Documents cités:
- WO-A1-2004/084753
- WO-A1-2007/060292
- US-A1- 2009 090 763

## Description

La présente demande de brevet concerne un dispositif d'interfaçage entre un utilisateur et un instrument chirurgical ou dentaire. Plus précisément, le présent mémoire concerne un dispositif d'interfaçage amovible et polyvalent.

L'environnement au sein d'un cabinet dentaire a considérablement évolué ces dernières années. En effet, soucieux de proposer à leurs patients des services toujours plus complets dans un cadre sécurisant et moderne, les praticiens investissent dans de l'équipement de plus en plus évolué. De plus, face à la diversité sans cesse croissante des interventions cliniques (restauration, endodontie, prophylaxie, orthodontie, implantologie etc.) auxquelles les praticiens se livrent, le nombre nécessaire d'instruments a considérablement augmenté.

Or, l'espace de travail dont dispose le praticien est limité. Tous les instruments nécessaires à une intervention doivent être groupés autour du patient assis sur le fauteuil et doivent rester à portée de main du praticien. Par ailleurs, les instruments doivent pouvoir être changés rapidement au cours de l'intervention et entre deux patients successifs. De même, les instruments doivent pouvoir être utilisés de manière simple et dans des conditions d'hygiène irréprochables afin de réduire le plus possible les risques d'erreurs de manipulation et de contamination.

Pour répondre à ces nouveaux besoins, chaque fabricant d'équipements médicaux apporte sa propre réponse en essayant de se différencier de ses concurrents. Pour cela, les fabricants créent des environnements de travail qui se distinguent de ceux de la concurrence tant du point de vue matériel (hardware) que du point de vue logiciel (software). Les fabricants vont par exemple proposer une interface utilisateur avec une nouvelle présentation graphique, un écran tactile ou la possibilité de choisir la langue d'utilisation.

Face à une telle abondance de l'offre, les praticiens ne parviennent le plus souvent pas à être suffisamment familiers de l'environnement de travail propre à chaque fabricant pour pouvoir utiliser les nombreux appareils dont ils disposent de façon intuitive, ce qui peut être source de risques tant pour les praticiens que pour leurs patients.

Pour remédier à ces problèmes, la tendance observée depuis plusieurs années est de regrouper sur une même unité d'alimentation et de commande (couramment appelée "unit") le plus grand nombre possible d'instruments afin de pouvoir utiliser l'interface utilisateur de l'unit pour commander l'ensemble de ces instruments. Cependant, les moyens de commande de l'unit ne disposent pas toujours de la flexibilité nécessaire pour permettre l'exploitation de toutes les fonctionnalités des instruments. Par ailleurs, les praticiens souhaitent souvent installer sur leur unit des instruments supplémentaires parfois bien après l'achat de l'unit. Dans ce cas de figure également, la facilité d'utilisation et la polyvalence de l'interface utilisateur qui équipe l'unit sont des facteurs-clés pour une intégration réussie et sans risque de nouveaux instruments dans l'unit.

Dans certains cas cependant, la modernisation de l'unit n'est pas possible et le praticien doit faire l'acquisition d'appareils de table en plus des instruments intégrés dans l'unit dentaire. Le nombre d'interfaces utilisateur que le praticien doit gérer a alors tendance à augmenter, ce qui peut représenter un risque pour le praticien ou le patient, ou tout du moins un manque d'efficacité.

Le document WO2007/060292 divulgue un appareil utilisé dans un environnement de soins dentaires et un procédé de commande d'un dispositif qui fait partie de l'appareil, en particulier pour la commande au niveau hygiénique et ergonomique d'un dispositif qui fait partie de l'appareil dans le cadre d'opérations de soins dentaires. L'appareil comprend au moins un dispositif utilisé dans le cadre des opérations de soins dentaires, par exemple une unité dentaire, une chaise de patient, un dispositif dentaire à rayons X et/ou un ordinateur, et au moins une interface d'utilisateur qui envoie des signaux de contrôle à au moins un desdits dispositifs utilisés dans le cadre des opérations de soins dentaires. Un moyen fixe ou amovible est disposé dans ladite interface d'utilisateur de manière à la relier de manière amovible à une personne qui travaille dans l'environnement de soins dentaires, à ses vêtements ou à l'unité dentaire. L'interface d'utilisateur peut également être conçue sous forme d'une pièce de vêtement fixe ou amovible disposée pour être portée séparément par la personne en question ou sur une autre structure à habiller.

On constate également aujourd'hui la création de plus en plus de cliniques dentaires dans lesquelles plusieurs praticiens travaillent en équipe et associent leurs spécialisations cliniques afin de proposer aux patients un service complet. De telles cliniques dentaires permettent également aux praticiens de partager tant les frais de fonctionnement (personnel assistant, loyer etc.) que les frais d'investissement.

Cependant, malgré le partage des installations, chaque praticien souhaite continuer à travailler selon ses habitudes. Il apparaît ainsi un besoin en outils de travail polyvalents qui soient utilisables de façon extrêmement flexible par plusieurs personnes. L'équipement, en particulier les units dentaires et les appareils de table, doivent donc pouvoir être adaptés aux besoins de chaque utilisateur.

La présente invention a pour but de répondre à ce besoin ainsi qu'à d'autres encore en procurant un dispositif d'interfaçage entre un utilisateur et un instrument chirurgical ou dentaire qui permet notamment à un utilisateur de recréer l'environnement de travail auquel il est habitué dans un contexte de partage des installations entre plusieurs utilisateurs.

A cet effet, la présente invention concerne un ensemble comprenant un dispositif d'interfaçage homme-machine portable et un contrôleur d'au moins un instrument chirurgical ou dentaire, le dispositif d'interfaçage homme-machine étant connecté de manière amovible au contrôleur qui est installé dans une unité d'alimentation et de commande ou un appareil de table, le dispositif d'interfaçage comprenant les moyens matériels et logiciels permettant à un utilisateur d'introduire dans le dispositif d'interfaçage une instruction ou une donnée pour contrôler, via le contrôleur, le fonctionnement de l'instrument chirurgical ou dentaire, le dispositif d'interfaçage comprenant l'ensemble des programmes nécessaires à l'utilisation de l'instrument chirurgical ou dentaire connecté à l'unité d'alimentation et de commande ou à l'appareil de table ainsi que le protocole de communication entre le dispositif d'interfaçage et le contrôleur, le dispositif d'interfaçage et le contrôleur formant un ensemble maître/esclave dans lequel le dispositif d'interfaçage est le maître et le contrôleur est l'esclave.

Grâce à ces caractéristiques, la présente invention procure un dispositif d'interfaçage homme-machine amovible grâce auquel l'utilisateur peut recréer l'univers de travail auquel il est habitué par simple connexion du dispositif d'interfaçage avec le contrôleur des instruments chirurgicaux ou dentaires que l'utilisateur souhaite employer. Le caractère portatif et amovible du dispositif d'interfaçage selon l'invention permet donc à son utilisateur d'avoir toujours à sa disposition, quel que soit le lieu où il se trouve, les programmes d'utilisation correspondant chacun à un instrument différent. Par ailleurs, le caractère amovible du dispositif d'interfaçage permet à plusieurs utilisateurs de partager les mêmes installations, chaque utilisateur étant muni de son propre dispositif d'interfaçage qui lui permet de travailler selon ses habitudes.

Selon une façon particulière de réalisation de l'invention, le dispositif d'interfaçage comprend les moyens matériels et logiciels qui lui permettent de traiter les informations provenant du contrôleur de l'instrument chirurgical ou dentaire et d'envoyer en retour au contrôleur les instructions correspondantes.

Selon encore une façon particulière de réalisation de l'invention, le dispositif d'interfaçage comprend les moyens matériels et logiciels qui lui permettent de traiter et de mémoriser les informations provenant du contrôleur de l'instrument chirurgical ou dentaire et d'exporter ces informations vers une base de données résidante ou distante.

Selon encore une façon particulière de réalisation de l'invention, le dispositif d'interfaçage comprend au moins un clavier permettant à l'utilisateur d'introduire une instruction ou une donnée et au moins un moyen d'affichage d'une information.

D'autres caractéristiques et avantages de la présente invention ressortiront plus clairement de la description détaillée qui suit d'un mode de réalisation du dispositif d'interfaçage homme-machine selon l'invention, cet exemple étant donné à titre purement illustratif et non limitatif seulement en liaison avec le dessin annexé sur lequel:
- les figures 1A et 1B illustrent schématiquement la connexion entre un dispositif d'interfaçage selon l'invention et une unité d'alimentation et de commande, respectivement un appareil de table;
- la figure 2 illustre schématiquement la connexion entre plusieurs dispositifs d'interfaçage selon l'invention et une même unité d'alimentation et de commande, et
- la figure 3 illustre schématiquement une unité d'alimentation et de commande équipée d'un socle pour la réception d'un dispositif d'interfaçage selon l'invention.

La présente invention est partie du constat que les praticiens, dentistes ou chirurgiens, sont amenés à utiliser un nombre toujours plus important d'instruments chirurgicaux ou dentaires dans la pratique quotidienne de leur profession. Pour faire face à une telle situation, la tendance est d'équiper les unités d'alimentation et de commande (couramment appelées "unit") d'un nombre le plus grand possible d'instruments afin de pouvoir utiliser l'interface utilisateur de l'unit pour commander l'ensemble de ces instruments. Cependant, les moyens de commande de l'unit ne disposent pas toujours de la flexibilité nécessaire pour permettre l'exploitation de toutes les fonctionnalités des instruments. C'est pourquoi les praticiens doivent souvent faire l'acquisition d'instruments additionnels que l'on appelle communément appareils de table. Toutefois, l'utilisation conjointe des instruments de l'unit et des appareils de table ne va pas toujours sans poser de problème au praticien. Il n'est en effet pas rare que l'environnement utilisateur des instruments de l'unit et des appareils de table ne soient pas les mêmes. Les praticiens doivent donc constamment jongler avec des environnements ou interfaces utilisateurs différents qui ne leur sont pas tous aussi familiers les uns que les autres. Cela implique donc pour les praticiens une charge de travail supplémentaire qui représente un risque pour les praticiens eux-mêmes ou pour leurs patients. Par ailleurs, avec le développement des cliniques dentaires qui regroupent plusieurs praticiens, se pose le problème de l'utilisation partagée d'installations communes. Il est en effet souhaitable que chaque praticien, succédant à l'un de ses confrères, puisse retrouver aussi rapidement que possible l'environnement utilisateur auquel il est habitué. Or, dans l'état actuel de la technique, cela ne peut se faire sans de nombreux réglages préalables qui représentent une perte de temps et sont risques d'erreurs. Il existait donc dans l'état de la technique un besoin pour un dispositif procurant une interface homme-machine universelle permettant d'utiliser toute une panoplie d'instruments dans un environnement contextuel identique et transportable d'une installation à une autre installation.

Une façon particulière de réalisation de la présente invention a pour but de répondre à cette attente en procurant un dispositif d'interfaçage homme-machine portable destiné à être connecté de manière amovible avec un contrôleur d'au moins un instrument chirurgical ou dentaire.

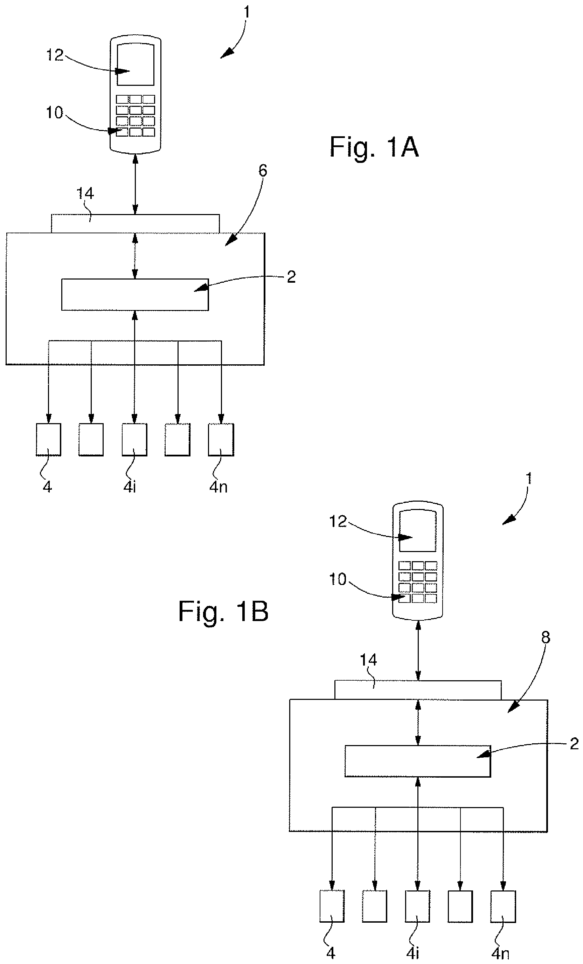

Désigné dans son ensemble par la référence numérique générale 1 (voir figure 1A), ce dispositif d'interfaçage comprend les moyens matériels ("hardware" en terminologie anglo-saxonne) et les moyens logiciels ("software" en terminologie anglo-saxonne) permettant à un utilisateur d'introduire dans le dispositif d'interfaçage 1 selon l'invention une instruction ou une donnée pour contrôler, via un contrôleur 2, le fonctionnement d'un instrument chirurgical ou dentaire choisi parmi une pluralité d'instruments 4,..., 4i,..., 4n.

Par contrôleur, on entend un système qui regroupe les circuits électroniques de puissance pour l'alimentation en courant électrique d'au moins un instrument chirurgical ou dentaire 4 ainsi que les programmes algorithmiques de régulation du fonctionnement de l'instrument 4. Par construction, le contrôleur 2 est un système sédentaire qui est renfermé dans le bâti d'une unité d'alimentation et de commande 6 (encore appelée "unit") ou d'un appareil de table 8.

Le contrôleur 2 contrôle le fonctionnement d'au moins un instrument chirurgical ou dentaire 4. Par instrument, on entend de manière non limitative un ou plusieurs moteurs, des pièces à main, des contre-angles, des turbines, des détartreurs, des scies électriques et autres. Ces instruments peuvent être connectés à l'unit 6 ou à l'appareil de table 8.

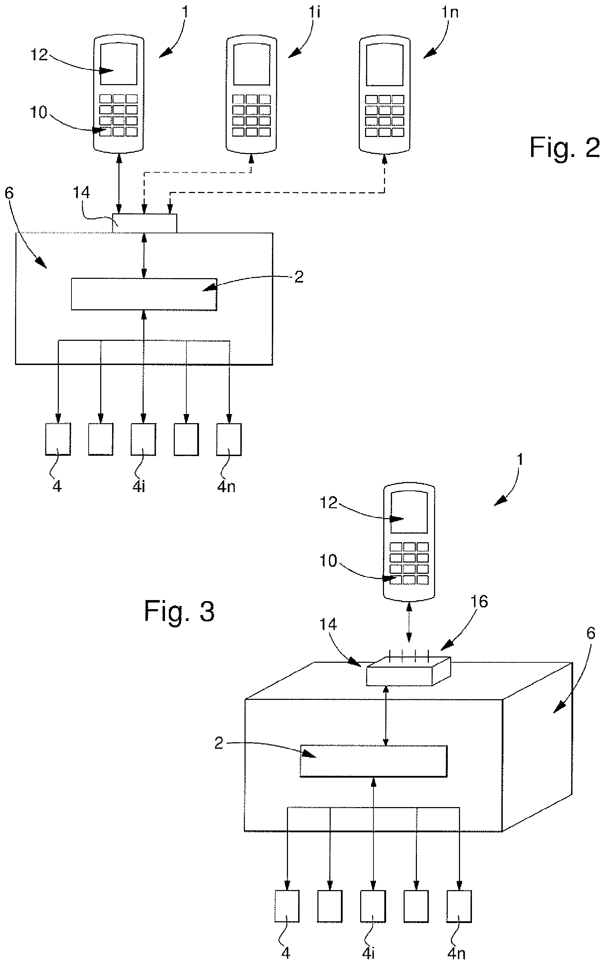

Selon une façon particulière de réalisation de l'invention, un même dispositif d'interfaçage 1 permet, via un même contrôleur 2 intégré par exemple dans une unit 6, de contrôler plusieurs instruments chirurgicaux ou dentaires 4, ..., 4i,..., 4n. Par ailleurs, un même dispositif d'interfaçage 1 peut être relié à des contrôleurs 2 différents intégrés par exemple dans une unit 6 et dans un appareil de table 8 (voir figure 1B). Enfin, plusieurs dispositifs d'interfaçage 1,..., 1i,..., 1n appartenant à des utilisateurs différents peuvent être reliés au même contrôleur 2 (voir figure 2).

Pour permettre à un utilisateur de contrôler le fonctionnement d'un instrument chirurgical ou dentaire 4 donné, le dispositif d'interfaçage 1 selon une forme de réalisation particulière de l'invention comprend au moins un clavier 10 permettant à l'utilisateur d'introduire une instruction ou une donnée et au moins un moyen d'affichage d'une information 12. Selon un premier mode de réalisation, le clavier 10 est un clavier à touches réelles combiné avec un écran d'affichage par exemple du type à cristal liquide. Selon un second mode de réalisation, le clavier 10 et le dispositif d'affichage 12 sont combinés dans un écran tactile qui inclut un clavier virtuel et un écran d'affichage.

Un dispositif d'interfaçage 1 donné est relié au contrôleur 2 par exemple d'une unit 6 via une liaison câblée ou une liaison sans-fil. Dans le cas d'une liaison câblée entre le dispositif d'interfaçage 1 et le contrôleur 2, cette liaison peut se faire via un socle 14 posé sur ou intégré de façon fixe dans l'unit 6 à un emplacement prévu à cet effet. Le fait de poser le dispositif d'interfaçage 1 sur son socle 14 assure la stabilité mécanique du dispositif d'interfaçage 1 et permet d'établir automatiquement la connexion électrique entre le dispositif d'interfaçage 1 et le contrôleur 2 de l'unit 6. La connexion électrique peut se faire via un connecteur électrique spécifique 16 (voir figure 3).

On notera qu'en fonction du type de dispositif d'interfaçage 1 utilisé et de son boîtier, le socle 14 prévu pour recevoir le dispositif d'interfaçage 1 peut varier, se distinguant par exemple par la géométrie de l'emplacement dans lequel vient se loger le dispositif d'interfaçage 1, l'emplacement du connecteur ou encore le design (couleur, forme). On évite ainsi tout risque de mauvaise utilisation du dispositif d'interfaçage 1.

Le dispositif d'interfaçage 1 selon l'invention peut être un dispositif disponible sur le marché grand-public comme par exemple un smartphone du type i-Phone® ou tout autre appareil doté d'un clavier et d'un écran comme décrit ci-dessus et ayant la possibilité de communiquer avec l'environnement extérieur via une liaison câblée ou une liaison sans-fil.

Le dispositif d'interfaçage 1 selon l'invention peut communiquer avec le contrôleur 2 de l'instrument chirurgical ou dentaire 4 via un protocole de communication digital. Selon une forme particulière de réalisation de l'invention, le choix s'est arrêté sur un bus de communication série RS-232, principalement pour des raisons de simplicité de mise en œuvre et de sécurité d'utilisation. Il va néanmoins de soi que d'autres protocoles de communication comme Ethernet, USB, CAN, RS-485, tout type de bus terrain ou encore des protocoles de communication sans fil comme bluetooth sont également envisageables.

L'alimentation du dispositif d'interfaçage 1 selon l'invention peut se faire via le contrôleur 2, lui-même alimenté par l'unit 6 ou l'appareil de table 8 dans lequel il est implanté. Selon une variante, le dispositif d'interfaçage 1 peut fonctionner au moyen de piles ou de batteries rechargeables qui peuvent être rechargées par le contrôleur 2 implanté dans l'unit 6 ou bien encore au moyen d'un chargeur séparé. Enfin, il peut être envisagé de connecter directement le dispositif d'interfaçage 1 sur le réseau d'alimentation en électricité.

Conformément à une forme particulière de réalisation de l'invention, le dispositif d'interfaçage 1 comprend les moyens matériels (encore appelés "hardware") et les moyens logiciels (encore appelés "software") qui permettent de traiter les informations fournies par l'utilisateur et le contrôleur 2 et d'envoyer les instructions au contrôleur 2 et au dispositif d'affichage 12. Le dispositif d'interfaçage 1 et le contrôleur 2 forment ainsi un ensemble maître/esclave dans lequel le dispositif d'interfaçage 1 est le maître et le contrôleur 2 est l'esclave.

Les moyens logiciels implémentés dans le dispositif d'interfaçage 1 comprennent donc notamment l'ensemble des programmes nécessaires à l'utilisation de l'instrument chirurgical ou dentaire 4 connecté à l'unit 6 ou à l'appareil de table 8 ainsi que le protocole de communication entre le dispositif d'interfaçage 1 et le contrôleur 2. Plusieurs programmes d'utilisation correspondant chacun à un instrument différent peuvent être implantés dans le dispositif d'interfaçage 1. Alternativement, un même logiciel inclut les programmes d'utilisation de plusieurs instruments.

Les programmes informatiques d'utilisation des différents instruments 4 peuvent être chargés dans le dispositif d'interfaçage 1 selon l'invention depuis un ordinateur personnel au moyen d'un connecteur intégré, via une liaison sans fil ou bien encore au moyen d'une clé USB que l'on connecte directement sur le dispositif d'interfaçage 1. On peut également envisager de télécharger les programmes depuis un site Internet. D'autres applications informatiques permettant par exemple de mémoriser et de traiter les données récoltées lors des interventions chirurgicales afin de constituer des bases de données patients ou de gérer un agenda peuvent aussi être installés dans le dispositif d'interfaçage 1.

A l'aide des mêmes canaux de transmission que ceux mentionnés ci-dessus, on peut envisager d'exporter les données renfermées dans un dispositif d'interfaçage 1 vers un serveur central ou encore de synchroniser les données de deux tels dispositifs d'interfaçage.

Il va de soi que la présente invention n'est pas limitée au mode de réalisation qui vient d'être décrit et que diverses modifications et variantes simples peuvent être envisagées par l'homme du métier sans sortir du cadre de l'invention tel que défini par les revendications annexées à la présente demande de brevet. On notera que pour des raisons de sécurité, il est déconseillé d'ouvrir d'autres applications que celles nécessaires à l'utilisation des instruments prévus pour l'intervention.

## Revendications

1. Ensemble comprenant un dispositif d'interfaçage homme-machine portable et un contrôleur (2) d'au moins un instrument chirurgical ou dentaire (4), le dispositif d'interfaçage homme-machine étant connecté de manière amovible au contrôleur (2) qui est installé dans une unité d'alimentation et de commande (6) ou un appareil de table (8), le dispositif d'interfaçage (1) comprenant les moyens matériels et logiciels permettant à un utilisateur d'introduire dans le dispositif d'interfaçage (1) une instruction ou une donnée pour contrôler, via le contrôleur (2), le fonctionnement de l'instrument chirurgical ou dentaire (4), le dispositif d'interfaçage comprenant l'ensemble des programmes nécessaires à l'utilisation de l'instrument chirurgical ou dentaire (4) connecté à l'unité d'alimentation et de commande (6) ou à l'appareil de table (8) ainsi que le protocole de communication entre le dispositif d'interfaçage (1) et le contrôleur (2), le dispositif d'interfaçage (1) et le contrôleur (2) formant un ensemble maître/esclave dans lequel le dispositif d'interfaçage (1) est le maître et le contrôleur (2) est l'esclave.

2. Ensemble selon la revendication 1, **caractérisé en ce que** le dispositif d'interfaçage (1) comprend les moyens matériels et logiciels qui lui permettent de traiter les informations provenant du contrôleur (2) de l'instrument chirurgical ou dentaire (4) et d'envoyer en retour au contrôleur (2) les instructions correspondantes.

3. Ensemble selon l'une quelconque des revendications 1 ou 2, **caractérisé en ce que** le dispositif d'interfaçage (1) comprend les moyens matériels et logiciels qui lui permettent d'afficher les informations provenant du contrôleur (2) de l'instrument chirurgical ou dentaire (4) ainsi que les instructions ou données introduites par l'utilisateur.

4. Ensemble selon l'une quelconque des revendications 1 à 3 **caractérisé en ce que** le dispositif d'interfaçage (1) comprend les moyens matériels et logiciels qui lui permettent de traiter et de mémoriser les informations provenant du contrôleur (2) de l'instrument chirurgical ou dentaire (4) et d'exporter ces informations vers une base de données résidante ou distante.

5. Ensemble selon l'une quelconque des revendications 1 à 4, **caractérisé en ce que** plusieurs programmes d'utilisation correspondant chacun à un instrument (4) différent sont implantés dans le dispositif d'interfaçage (1).

6. Ensemble selon l'une quelconue des revendications 1 à 4, **caractérisé en ce qu'**un même logiciel inclut les programmes d'utilisation de plusieurs instruments (4).

7. Ensemble selon l'une quelconque des revendications 1 à 6, **caractérisé en ce que** le dispositif d'interfaçage (1) comprend au moins un clavier (10) permettant à l'utilisateur d'introduire une instruction ou une donnée et au moins un moyen d'affichage d'une information (12).

8. Ensemble selon l'une quelconque des revendications 1 à 7, **caractérisé en ce que** le dispositif d'interfaçage (1) est relié au contrôleur (2) via une liaison câblée ou une liaison sans fil.

9. Ensemble selon la revendication 8, **caractérisé en ce que**, dans le cas d'une liaison câblée entre le dispositif d'interfaçage (1) et le contrôleur (2), la liaison se fait via un socle (14) posé sur ou intégré de façon fixe dans l'unité d'alimentation et de commande (6) ou l'appareil de table (8).

10. Ensemble selon l'une quelconque des revendications 1 à 9, **caractérisé en ce que** le dispositif d'interfaçage (1) et le contrôleur (2) de l'instrument chirurgical ou dentaire (4) sont reliés ensemble au moyen d'un bus de communication série RS-232.

11. Ensemble selon l'une quelconque des revendications 1 à 10, **caractérisé en ce que** l'alimentation du dispositif d'interfaçage (1) se fait via le contrôleur (2), le contrôleur (2) étant alimenté par l'unité d'alimentation et de commande (6) ou l'appareil de table (8) dans lequel le contrôleur (2) est implanté.

12. Ensemble selon l'une quelconque des revendications 1 à 10, **caractérisé en ce que** le dispositif d'interfaçage (1) fonctionne au moyen de piles ou de batteries rechargeables qui sont rechargées par le contrôleur (2) implanté dans l'unité d'alimentation et de commande (6) ou l'appareil de table (8) ou qui sont rechargées au moyen d'un chargeur séparé.

13. Ensemble selon l'une quelconque des revendications 1 à 12, **caractérisé en ce que** les logiciels d'utilisation des instruments (4) sont chargeables dans le dispositif d'interfaçage (1) depuis un ordinateur personnel au moyen d'un connecteur intégré, via une liaison sans fil ou au moyen d'une clé USB.

## Patentansprüche

1. Anordnung, umfassend eine tragbare Mensch-Maschine-Schnittstellenvorrichtung und eine Steuerung (2) für mindestens ein chirurgisches oder zahnmedizinisches Instrument (4), wobei die Mensch-Maschine-Schnittstellenvorrichtung trennbar mit der Steuerung (2) verbunden ist, die in einer Stromversorgungs- und Steuereinheit (6) oder einem Tischgerät (8) installiert ist, wobei die Schnittstellenvorrichtung (1) die Hard- und Softwaremittel umfasst, die es einem Benutzer ermöglichen, in die Schnittstellenvorrichtung (1) eine Anweisung oder Daten einzugeben, um den Betrieb des chirurgischen oder zahnmedizinischen Instruments (4) über die Steuerung (2) zu steuern, wobei die Schnittstellenvorrichtung alle Programme, die erforderlich sind, um das chirurgische oder zahnmedizinische Instrument (4), das mit der Stromversorgungs- und Steuereinheit (6) oder dem Tischgerät (8) verbunden ist, zu benutzen, sowie das Kommunikationsprotokoll zwischen der Schnittstellenvorrichtung (1) und der Steuerung (2) umfasst, wobei die Schnittstellenvorrichtung (1) und die Steuerung (2) zusammen eine Master-Slave-Anordnung bilden, bei der die Schnittstellenvorrichtung (1) der Master und die Steuerung (2) der Slave ist.

2. Anordnung nach Anspruch 1, **dadurch gekennzeichnet, dass** die Schnittstellenvorrichtung (1) die Hard- und Softwaremittel umfasst, die es ihr ermöglichen, die Informationen von der Steuerung (2) des chirurgischen oder zahnmedizinischen Instruments (4) zu verarbeiten und die entsprechenden Anweisungen an die Steuerung (2) zurückzusenden.

3. Anordnung nach einem der Ansprüche 1 oder 2, **dadurch gekennzeichnet, dass** die Schnittstellenvorrichtung (1) die Hard- und Softwaremittel umfasst, die es ihr ermöglichen, die Informationen von der Steuerung (2) des chirurgischen oder zahnmedizinischen Instruments (4) sowie die vom Benutzer eingegebenen Anweisungen oder Daten anzuzeigen.

4. Anordnung nach einem der Ansprüche 1 bis 3, **dadurch gekennzeichnet, dass** die Schnittstellenvorrichtung (1) die Hard- und Softwaremittel umfasst, die es ihr ermöglichen, die Informationen von der Steuerung (2) des chirurgischen oder zahnmedizinischen Instruments (4) zu verarbeiten und zu speichern und diese Informationen in eine lokale oder entfernte Datenbank zu exportieren.

5. Anordnung nach einem der Ansprüche 1 bis 4, **dadurch gekennzeichnet, dass** mehrere Anwendungsprogramme, die jeweils einem unterschiedlichen Instrument (4) entsprechen, in der Schnittstellenvorrichtung (1) installiert sind.

6. Anordnung nach einem der Ansprüche 1 bis 4, **dadurch gekennzeichnet, dass** die gleiche Software die Anwendungsprogramme mehrerer Instrumente (4) umfasst.

7. Anordnung nach einem der Ansprüche 1 bis 6, **dadurch gekennzeichnet, dass** die Schnittstellenvorrichtung (1) mindestens eine Tastatur (10), die es dem Benutzer ermöglicht, eine Anweisung oder Daten einzugeben, sowie mindestens ein Mittel zum Anzeigen von Informationen (12) umfasst.

8. Anordnung nach einem der Ansprüche 1 bis 7, **dadurch gekennzeichnet, dass** die Schnittstellenvorrichtung (1) über eine Kabelverbindung oder eine drahtlose Verbindung mit der Steuerung (2) verbunden ist.

9. Anordnung nach Anspruch 8, **dadurch gekennzeichnet, dass** im Fall einer Kabelverbindung zwischen der Schnittstelleneinrichtung (1) und der Steuerung (2) die Verbindung über eine Basis (14) erfolgt, die fest an der Stromversorgungs- und Steuereinheit (6) oder dem Tischgerät (8) montiert ist oder in diese integriert ist.

10. Anordnung nach einem der Ansprüche 1 bis 9, **dadurch gekennzeichnet, dass** die Schnittstellenvorrichtung (1) und die Steuerung (2) des chirurgischen oder zahnmedizinischen Instruments (4) über einen seriellen RS-232-Kommunikationsbus miteinander verbunden sind.

11. Anordnung nach jeglichem der Ansprüche 1 bis 10, **dadurch gekennzeichnet, dass** die Stromversorgung der Schnittstellenvorrichtung (1) über die Steuerung (2) erfolgt, wobei die Steuerung (2) durch die Stromversorgungs- und Steuerungseinheit (6) oder das Tischgerät (8), in das die Steuerung (2) eingebaut ist, mit Strom versorgt wird.

12. Anordnung nach einem der der Ansprüche 1 bis 10, **dadurch gekennzeichnet, dass** die Schnittstellenvorrichtung (1) mittels Batterien oder wiederaufladbaren Batterien arbeitet, die durch die in die Stromversorgungs- und Steuereinheit (6) oder das Tischgerät (8) eingebauten Steuerung (2) oder mit Hilfe eines separaten Ladegeräts wieder aufgeladen werden.

13. Anordnung nach einem der Ansprüche 1 bis 12, **dadurch gekennzeichnet, dass** die Anwendungssoftware der Instrumente (4) von einem Personal Computer über einen eingebauten Anschluss mittels einer kabellosen Verbindung oder einem USB-Stick auf die Schnittstellenvorrichtung (1) geladen werden kann.

## Claims

1. Assembly comprising a portable human-machine interface device and a controller (2) of at least one surgical or dental instrument (4), the human-machine interface device being removably connected to the controller (2) which is installed in a power and control unit (6) or in a table top equipment (8), the interface device (1) including the hardware and software means allowing a user to enter an instruction or data into the interface device (1) to control, via the controller (2), the operation of the surgical or dental instrument (4), the interface device comprising all the programmes necessary for using the surgical or dental instrument (4) connected to the power and control unit (6) or to the table top equipment (8) in addition to the communication protocol between the interface device (1) and the controller (2), the interface device (1) and the controller (2) form a master/slave assembly in which the interface device (1) is the master and the controller (2) is the slave.

2. Assembly according to claim 1, **characterized in that** the interface device (1) includes hardware and software means for processing the information from the controller (2) of the surgical or dental instrument (4) and for sending back the corresponding instructions to the controller (2).

3. Assembly according to any of claims 1 or 2, **characterized in that** the interface device (1) includes hardware and software means for displaying the information from the controller (2) of the surgical or dental instrument (4) and the instructions or data entered by the user.

4. Assembly according to any of claims 1 to 3, **characterized in that** the interface device (1) includes hardware and software means for processing and storing the information from the controller (2) of the surgical or dental instrument (4) and for exporting said information to a resident or remote data base.

5. Assembly according to any of claims 1 to 4, **characterized in that** several user programmes each corresponding to a different instrument (4) are implemented in the interface device (1).

6. Assembly according to any of claims 1 to 4, **characterized in that** the same software includes the user programmes for several instruments (4).

7. Assembly according to any of claims 1 to 6, **characterized in that** the interface device (1) includes at least one keyboard (10) allowing the user to enter an instruction or data and at least one means of displaying information (12).

8. Assembly according to any of claims 1 to 7, **characterized in that** the interface device (1) is connected to the controller (2) via a wired connection or a wireless connection.

9. Assembly according to claim 8, **characterized in that**, in the case of a wired connection between the interface device (1) and the controller (2), the connection occurs via a base (14) placed on or fixedly integrated in the power and control unit (6) or the table top equipment (8).

10. Assembly according to any of claims 1 to 9, **characterized in that** the interface device (1) and the controller (2) of the surgical or dental instrument (4) are connected to each other by means of a RS-232 serial communication bus.

11. Assembly according to any of claims 1 to 10, **characterized in that** the interface device (1) is powered via the controller (2), the controller (2) being powered by the power and control unit (6) or the table top equipment (8) in which the controller is integrated.

12. Assembly according to any of claims 1 to 10, **characterized in that** the interface device (1) operates by means of ordinary batteries or rechargeable batteries which are recharged by the controller (2) integrated in the power and control unit (6) or the table top equipment (8) or which are recharged by means of a separate charger.

13. Assembly according to any of claims 1 to 12, **characterized in that** the software for using the instruments (4) can be loaded in the interface device (1) from a personal computer by means of an integrated connector, via a wireless connection or via a USB stick.
